# EUROPEAN PATENT APPLICATION

(11) **EP 4 666 959 A1**
(43) Date of publication of application: **24.12.2025**
(21) Application number: 24187809.9
(22) Date of filing: 10.07.2024
(51) Int. Cl.: A61B 10/00, A61B 5/00

(54) **DISPOSABLE DEVICE FOR TESTING PROPERTIES OF VAGINAL SECRETION**

(30) Priority: 20.06.2024 CN 202410810489; 20.06.2024 CN 202421422287 U
(71) Applicant: Zhejiang Orient Gene Biotech Co., Ltd, Anji Huzhou Zhejiang 313300 (CN)
(72) Inventor: ZHANG, Ying, Huzhou, 313300 (CN); FANG, Jianqiu, Huzhou, 313300 (CN)
(74) Representative: Piotrowicz, Pawel Jan Andrzej

(57) **Abstract**

The invention provides a disposable device for testing an analyte in a vaginal secretion, and the device includes: a smooth rod body configured to enter the vagina, where a test area is arranged on the smooth rod body, and the test area protrudes from a surface of the smooth rod body, such that when the smooth rod body enters the vagina, the protruding test area is capable to contact or receive a vaginal secretion to test a property of the vaginal secretion.

## Description

### CROSS-REFERENCE TO RELATED APPLICATIONS

This application claims priority to the Chinese Patent Application, Application No. CN2024108104892, filed on June 20, 2024, the Chinese Patent Application, Application No. CN2024214222872, filed on June 20, 2024, and all disclosures of the Applications, including but not limited to the specification, abstract, claims and accompanying drawings of this application are hereby made a part of this application.

### BACKGROUND OF THE INVENTION

### Field of the Invention

The invention relates to the field of in vitro detection, and in particular, to a disposable testing device for testing a vaginal secretion.

### Description of the Related Art

The following description is merely an introduction of some background knowledge and does not constitute any limitation to the invention.

The composition and physical characteristics of liquid are often used in test to understand the situation of the source of the liquid. For example, oxidants for testing biological liquids, pH value, specific gravity, creatinine, bilirubin, glucose, and other similar indicators can show health status or contamination. Food and beverage test items include pH value, specific gravity, contamination by bacteria or toxic substances, and the like. Test indicators of soil and water samples also include pH value, specific gravity, bacteria, lead or mercury contamination.

Generally, these rapid chemical tests can be accomplished by using test strips with a series of chemical test pads directly dipped with samples and reading test results directly, without using capillary action. The test pad chemically reacts with the test sample or sample components to cause a color change. Usually, an operator only needs to immerse the test strips in the liquid, then observe the color change of the test pad, and compare the color of the test pad with that of the result card to determine the test result.

However, such test strips of "dipping samples - reading results" has many disadvantages, and especially they cannot be combined with modern new rapid immunoassay devices. Nevertheless, these chemical tests are still necessary. The new rapid immunoassay devices are designed to reduce the contact opportunity between the operator and the samples under test. By using the testing method of "dipping samples - reading results", the operator must open the testing device, which will cause the samples to be contaminated by the operator or other reagents on the test strip.

For example, U.S. Pat. No. US7, 595, 196 mainly discloses a test strip, and a hydrophobic medium is arranged on the test strip and can prevent the liquid from flowing back from the test pad to an absorbent pad, thus causing the reverse flow of the liquid sample. Although this can be improved, in the existing traditional products, there is still the phenomenon that colored substances on reaction pads diffuse with the forward flow of the liquid. In addition, liquids among different test pads are mixed, which causes the interference of the test pads.

At present, a conventional testing device for testing a property of a vaginal secretion features in going deep into the vagina for sampling with a cotton swab and then coating the taken sample onto the test area for testing; therefor, operation is complex and needs to be done by experienced professionals. However, it is often desirable to test the property of the vaginal secretion at home, and this requires an improvement in the conventional testing device to facilitate test at home.

### BRIEF SUMMARY OF THE INVENTION

For the disadvantages of the above conventional technology, the invention provides a disposable device for testing a property of a vaginal secretion, and the device can conveniently test vaginal microenvironment and can be operated at home by users themselves; and such operation is simple and convenient and can be completed in one step.

Therefore, the invention provides a disposable device for testing a vaginal secretion, and the device includes: a smooth rod body configured to enter a vagina, where a test area is arranged on the smooth rod body, and the test area protrudes from a surface of the smooth rod body, such that when the smooth rod body enters the vagina, the protruding test area is capable to contact or receive the vaginal secretion to test the property of the vaginal secretion.

Generally, the smooth rod body is a curved rod body; for example, a curved rod body without sharp corners is easy to enter the vagina for sampling without damaging vaginal tissues. The smooth rod body is generally plastic injection molding, or may be smoothed by surface treatment after injection molding, such as absorbent materials; or it can be mechanically sandblasted to make the whole surface smooth without the sharp corners.

In some embodiments, the smooth rod body includes a chamber including a testing element therein, and the test area or a part of the test area protrudes from the surface of the rod body, and the protruding test area directly receives or contacts the vaginal secretion, such that the test can be directly performed on the test area. It can be understood that the testing element can be arranged in the chamber, and the test area thereon protrudes from the surface of the rod body, to facilitate contact with the vaginal secretion. Secretion here can be urine or liquid secreted by vagina, such as a mixture of fatty acids, and testing the property or state of these secretions or liquid secretions can evaluate the microenvironment of the vagina.

In some embodiments, the rod body is provided with a window where the test area protrudes, and it can be understood that the test area directly protrudes outward from the window instead of being located in the window, such that the test area is integrated with collection and testing functions, collection and testing can be completed in one step, and such operation is simple and convenient.

In some embodiments, the protruding areas of some test areas are in a radian form that is similar to an arch bridge form, such that they have a gradually rising curved surface, and can enter the vagina more smoothly without causing uncomfortable or unusual feelings. In some embodiments, the chamber includes a lug boss therein, and the test area is located on or covers the lug boss, such that the test area is located on the lug boss in a radian form. Alternatively, the test area or the part of the test area protrudes through the lug boss in a radian or curved surface form, especially at the window. In some embodiments, a protrusion is arranged below the window, and the test area protrudes from the position of the window and is higher than the surface of the smooth rod body. Thus, when the lug boss shares a height with the surface of the rod body, the test area is plotted in a curved surface form, and is a little higher than the plane. For example, a vertical distance from the highest position of the curved surface to the plane is 2-3 mm, and the distance decreases gradually from the highest position to the plane. In some embodiments, the test area is made of a flexible material, and is distanced from the surface of the lug boss by 2-3 mm, without completely covering the surface of the lug boss; therefore, when the test area enters the vagina together with the vagina, it has an upward or downward movement distance of 2-3 mm, and will not damage the vagina due to different operations. In some embodiments, the lug boss is a curved lug boss that protrudes outwardly from the window, and the test area covers the curved surface. In some embodiments, the lug boss is a right-angled cube lug boss, for example, a square lug boss and a cuboid lug boss. In fact, there is a plane in the lug boss, the plane is parallel to or share a height with the plane where the window is located. When covering the plane, the test area receives lateral and longitudinal pressure from the edge of the window, such that the test area slightly protrudes upward to form a curved protrusion similar to the arch bridge form, and the test area departs from the plane and is supported by the lateral edge of the plane.

In some embodiments, the rod body includes a lower card and an upper card, the lower card is provided with the lug boss, the upper card is provided with the window, and the lug boss is located below the window. In some embodiments, the lower card has a recessed area, the upper card is arranged in the recessed area and covers the recessed area, and a test strip is arranged between the upper card and the lower card. Equivalently, the upper card covers the whole recessed area, such that a rod-shaped object can exist in a flat form. Of course, the rod-shaped object may be cylindrical, and a front end of the rod-shaped object is curved without sharp corners. In some embodiments, the part of the test area is higher than the surface of the upper card, and preferably 1-5 mm higher than the surface.

In some embodiments, the upper card is provided with a limit block, and the limit block is used for limiting the front, back, left and right positions of the test strip in the recessed area of the lower card, such that the test strip is stably located in the chamber. In some embodiments, a plane of the lug boss along a longitudinal axis substantially shares a height or a plane with a surface of the upper card, an edge of the window is in contact with the test area to give the test area a pressure, and a stress point where the pressure is applied is lower than the plane. In some embodiments, the plane of the lug boss is a right-angled plane, and a height of the edge of the window is a thickness of the upper card. Therefore, when the upper card covers the recessed area, the test area receives the pressure from the edge of the window and is lower than the plane of the lug boss, such that the test area protrudes upward or bulge upward from the plane where the window is located. Alternatively, the lug boss has two edges, and the window has two edges, such that the test area protrudes upward from the lug boss under the pressure from the edge of the window, and the test area is arched on the curved surface under the pressure of the two edges of the lug boss and a pair of edges of the window, and the highest position of the curved surface is away from the surface of the lug boss.

In order to allow the test area to bulge or protrude, the test area is flexible. Therefore, in some embodiments, a test pad for testing water absorption capability is arranged on the test area, a chemical reagent is treated on the test area, and the chemical reagent is capable to react with the secretion and produce color change. In some embodiments, the test pad is made of absorbent material, and the surface of the absorbent material is smooth but not rough. In some embodiments, the absorbent material is flexible, and the flexible absorbent material can be stretched. In some embodiments, the absorbent material is used to treat the chemical reagent for testing a pH value. The absorbent material is arranged on a flexible non-absorbent backing. In some embodiments, one end of the non-absorbent backing is provided with an adhesive area, and the area is provided with an adhesive glue layer that is fixed to the upper card, while the end of the non-absorbent backing with the test area is a free end and not fixed.

The invention provides a method for testing a property of a vaginal secretion, and the method includes: providing the testing device; allowing a smooth rod-shaped body to be inserted into the vagina; taking out the rod-shaped body; observing the color change of the test area; and comparing the color with that in a standard color card, to judge the property of the secretion. The signal is compared with a standard signal. In some specific embodiments, a detectable signal may be the color change of the reaction pad or the testing pad. The "standard" may be any appropriate and objective way of expressing a test result. For example, the standard may be a standard comparison table or card provided for the device, or together with the device, or in other possible ways, and the comparison of the signal with that described in the standard includes the comparison of the color of the reaction pad after the test with the color of the standard table or card, and determining the test results through such comparison. The detectable signal may be indicative of any objective analytical result (for example, fluorescence, enzyme-based assay, or spectrophotometric assay). The standard can be made based on any of these or other testing methods.

The invention also provides a kit for testing a liquid sample. The kit includes the device of the invention and an operation instruction of the device. In different specific embodiments, the kit can also include an analysis result comparison table or other standards. The kit can be packaged in any suitable form, such as vacuum sealed boxes, plastic bags or aluminum foil bags.

The invention further includes some other useful aspects described in detail here. These aspects can be fully understood by using these products. These aspects need to be further combined with various specific embodiments for investigation to obtain complete evaluation in the invention. In addition, other aspects and specific embodiments of the invention will also be described in detail.

The summary of the invention is not limited to the above description, and other features and benefits of the invention are reflected from the following detailed description and claims.

### BRIEF DESCRIPTION OF THE DRAWINGS

FIG. 1 is a schematic diagram showing an exploded structure of a testing device according to a specific embodiment of the invention.

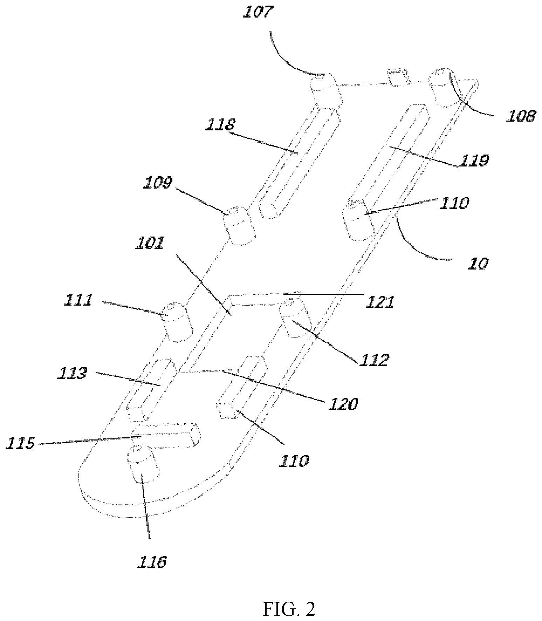

FIG. 2 is a schematic diagram showing a three-dimensional structure of an upper card or an upper cover of a testing device according to a specific embodiment of the invention.

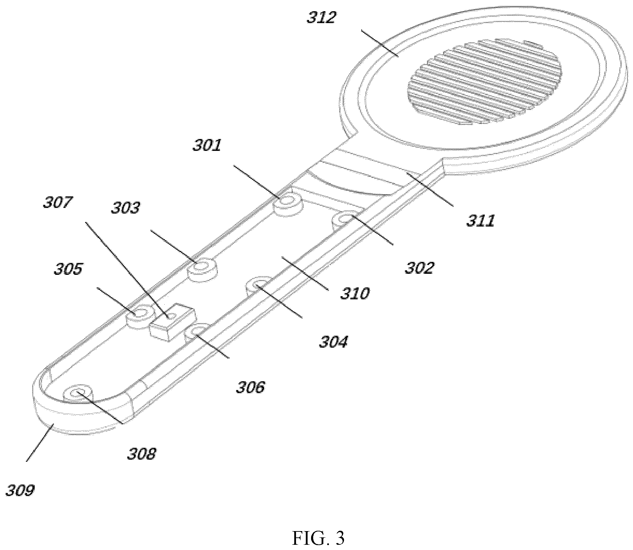

FIG. 3 is a schematic diagram showing a three-dimensional structure of a lower card of a testing device according to a specific embodiment of the invention.
FIG. 4 is a structural schematic diagram showing a back side of a testing device according to a specific embodiment of the invention.
FIG. 5 is a structural schematic diagram of a testing element according to a specific embodiment of the invention.
FIG. 6 is a structural schematic diagram of a testing element according to a specific embodiment of the invention.

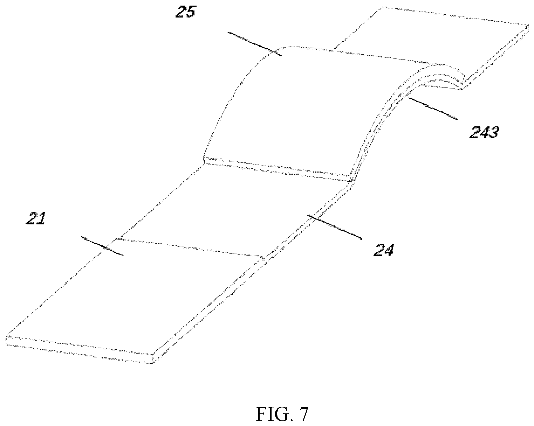

FIG. 7 is a structural schematic diagram of a testing element according to a specific embodiment of the invention.
FIG. 8 is a structural schematic diagram showing a positional relationship between a testing element and an upper cover according to a specific embodiment of the invention.
FIG. 9 is a structural schematic diagram showing combination of a testing element and an upper cover according to a specific embodiment of the invention.
FIG. 10 is a schematic diagram showing a three-dimensional structure of a testing device according to a specific embodiment of the invention.

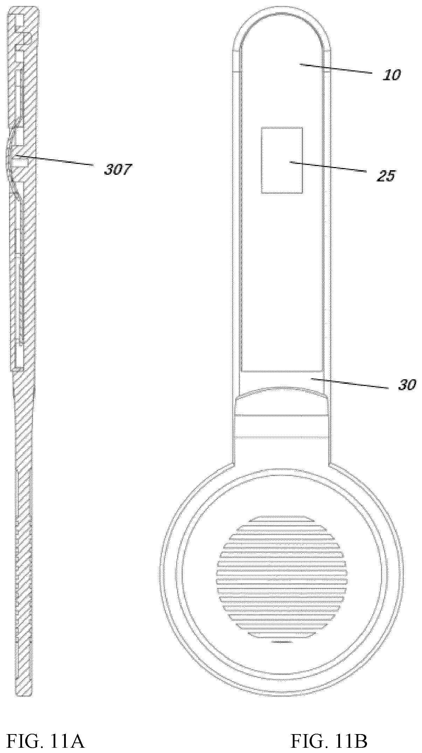

FIG. 11A is a structural schematic diagram showing a longitudinal section of a testing device shown in FIG. 11B according to a specific embodiment of the invention.
FIG. 12 is an enlarged diagram of a partial structure according to a specific embodiment of the invention.
FIG. 13 is a structural schematic diagram of combination of a test area with a lug boss and a window.

### DETAILED DESCRIPTION OF THE INVENTION

In the following detailed description, the accompanying drawings and corresponding textual descriptions are only intended to illustrate particular embodiments that are likely to be implemented in the invention. We do not exclude that the invention may also be implemented in other specific embodiments and that the structure of the invention may be altered without violating the application scope of the invention.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, detection means that the amount of a substance or material is tested. Further, assay also means immunoassay, chemical assay, enzyme assay, and the like. In a specific embodiment of the invention, a reaction pad or a test pad is used to test the property of a sample or the presence or absence of an analyte through chemical reaction, and the analyte can also be tested through an immune method.

### Liquid sample

Samples tested by the testing device of the invention include biological fluid (for example, case fluid or clinical sample). Liquid samples or fluid samples may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

The rod-shaped object can be directly inserted into the sample with the device of the invention, such that the test area can contact the liquid sample, the liquid sample is taken out, and the color change of the test area can be observed. Of course, the rod-shaped object 388 can be directly inserted into the vagina, such that the test area 25 or a part of the protruding test area can contact with liquid or receive vaginal secretions, so as to observe the color change of the test area and judge the presence or absence of an analyte or the property of the vaginal secretions, such as pH value. The liquid samples here also include female vaginal secretions.

### Female vaginal secretions

Vaginal secretions may be physiological leucorrhea, secretions during sexual arousal, or liquids or a mixture of the liquids and solids caused by vaginitis, cervicitis and endometritis. Therefore, any liquids produced or secreted in a female vagina falls with the analyte, or a part of the produced liquids become into the solids in a specific environment, and the mixture of the liquids and the solids also fall within the secretions of the invention. Physiological leucorrhea: female vaginal secretions include beneficial bacteria such as hyaluronic acid and lactobacillus, which can help maintain the acid-base balance of the vagina and prevent the invasion of pathogens. Before and after the ovulatory and menstrual period, vaginal secretions may increase due to changes in hormone levels, which is a normal physiological phenomenon. Secretions during sexual arousal: under sexual stimulation, the vagina will secrete the liquid that acts as a lubricant and helps the sexual life go smoothly. Vaginitis: vaginal inflammation may lead to symptoms such as increased vaginal secretions, color change, and peculiar smell. Common vaginitis includes bacterial vaginitis and vulvovnginal candidlasis. Cervicitis: cervical inflammation may lead to symptoms such as increased vaginal secretions, color change, and peculiar smell. Cervicitis may be caused by bacteria, viruses, chlamydia and other infections. Endometritis: endometrial inflammation may lead to increased vaginal secretions. Endometritis may be caused by bacterial infection and intrauterine device. In the invention, the test area directly contacts the vaginal secretions, so as to test some analytes or their properties, such as the pH value or the specific pH value, and to compare such pH values with the normal pH value and judge whether they fall within the normal range or the abnormal range.

### Downstream and upstream

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the testing device of the invention, when there is the liquid sample at the bottom of the testing chamber, for example, the liquid sample from the sample chamber, such as a urine sample or a saliva sample of a test subject, can flow from an end of the test strip to the other end of the test strip (that is to say, from the bottom to the top).

### Liquid communication

Liquid communication means that liquid or gas can flow from one place to another place. In a flow process, the liquid or gas may pass through some physical structures that play a guiding role. The "pass through some physical structures" here means that the liquid passes through the surfaces of these physical structures or their internal space and flows to another place passively or actively, where such passive flow is usually caused by external forces, such as flow under the capillary action and the action of air pressure. The flow here may be an active flow of the liquid due to self-action (gravity or pressure), or may be a passive flow therefore. The fluid may be a forward flow or also a reverse flow under the action of air pressure; or the fluid is caused to flow from a position to another position under the action of air pressure. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used for detecting drugs belonging to a medical use but easy to be taken excessively, such as tricyclic antidepressants (imipramine or analogues) and acetaminophen. These drugs are metabolized into micromolecular substances after being absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Any other clinical chemistry analysis may be performed by lateral flow test in combination with the device of the invention. The sample of the invention may be urine, and the analyte may be HCG, LH, and other substances, which are used for testing ovulation or early pregnancy.

The vagina has a relatively stable ecological balance. Affected by estrogens secreted by ovaries, vaginal epithelium changes periodically. After the vaginal epithelium sheds, intracellular glycogen is decomposed into lactic acid by lactobacillus in the vagina, such that the vagina is kept acidic. Lactobacilli are dominant in normal vaginal flora, such that the vaginal pH value is kept at 4.0-4.5. This is not conducive to the growth and reproduction of bacteria. As an estrogen level decreases after menopause, vaginal mucosa atrophy, epithelial thinning, exfoliated cells and glycogen reduction can cause an increased vaginal pH value. The vaginal pH value plays an important role in vaginal self-purification, and is one of the important mechanisms for human body to defend against vulvovaginal inflammation. Therefore, when vaginal flora is out of balance, the vaginal pH value changes accordingly. Therefore, testing the pH value of the vaginal secretion is an important indicator to measure the vaginal microenvironment.

### Testing element

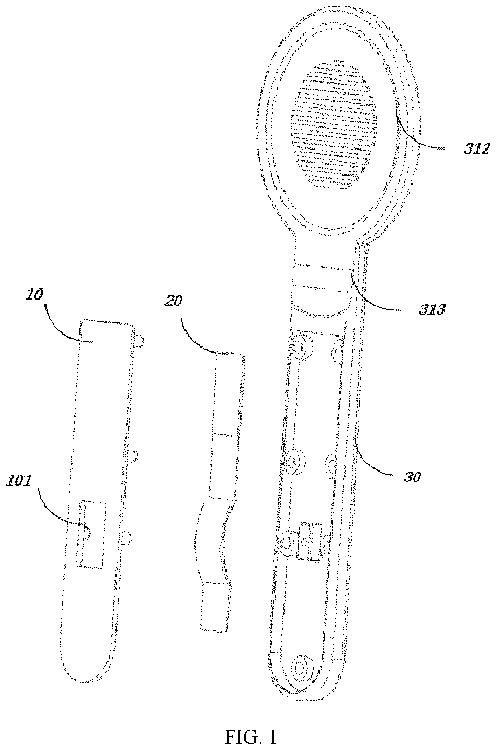

FIG. 1, FIG. 5 and FIG. 6 show a test strip 20 according to a specific embodiment of the invention. The test strip includes a test area 29 that is made of the absorbent material to form a test pad 29. The test strip has two ends, namely a first end 28 near the test area and a second end near the adhesive area 21. The test pad 25 is arranged on the test strip 20, and the test pad is made of the absorbent material. One non-absorbent area 23 or a section of the non-absorbent area 23 is arranged at the upstream of the test pad 25 made of the absorbent material, and one non-absorbent area 22 is also spaced at the downstream of the test pad. One adhesive area is arranged at the downstream of the non-absorbent area 22 with the glue layer thereon; the adhesive area is an area for fixing the test strip, such that when the test area does not contact with the lug boss, the test strip is straight and has no bend, and one end thereof is fixed. When the test area contacts with the lug boss, the test area is bent under the acting force of the lug boss and the straight line length of the test area becomes short. In this case, if one end of the test strip is fixed, the test area can be successfully bent. On the contrary, if the test strip is not fixed and at least one end of the test strip is not fixed, when the test area contacts with the lug boss, the test area is not easy to bend and remains straight because the lug boss has an upward force on the test area of the test strip. In this case, both ends of the test area contract or move at the same time, such that the test area is easy to bend. The test area includes the test pad 25 on which chemical substances are treated; the chemical substances can directly contact with the liquid sample to react, thereby causing the color change; the color change or the color shade is compared with that in the colourimetric card configured, so as to judge the presence or absence or concentration of the analyte, or the property of the liquid, such as the pH value.

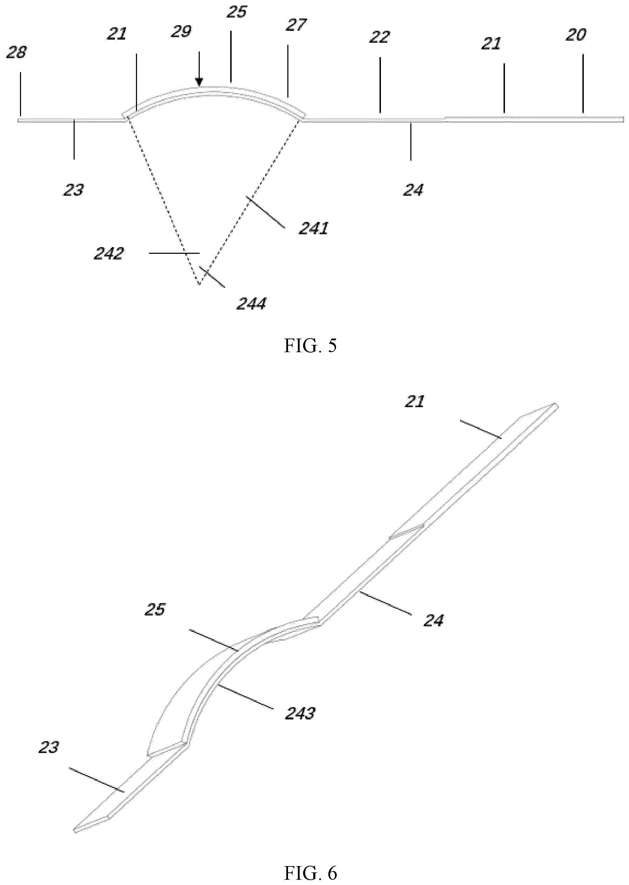

In some embodiments, a support 24 is further arranged to support the test pad 25, and serves as a backing; the support may be made of any approximate materials, for example, plastics, non-absorbent paper or paperboard, glass, metal or aluminum foil. The support 24 as a bottom board is arranged below the absorbent material, or the absorbent test pad 25 is bonded onto the surface of a part of the backing 243; therefore, the support is non-absorbent and provided with several areas thereon, and such area include the area where the test pad 25 is bonded, the adhesive area 21 arranged on the other end of the support, the non-absorbent area 23 nearby the front end 28 of the test pad 25 on the support, and the non-absorbent area 22 between the test pad 25 and the adhesive area. In some embodiments, the test pad 25 is made of the absorbent and malleable material, and its surface is a relatively smooth surface rather than a rough surface; therefore, when the test end enters the vagina, it can absorb the liquid sample and makes a test subject feel natural, without causing uncomfortable feeling, which increases the comfort level of the test. The test subject feels uncomfortable when foreign matters enter the vagina. Therefore, the surface of the testing device 388 (insertion end) entering the vagina is smooth as far as possible, and no sharp edges and corners appear on the insertion end; the whole surface of the testing device is smooth; the overall design of the testing device is streamlined; and the test area is also curved and forms the streamlined structure with the insertion end as a whole. Therefore, the test area or the part of the test pad 25 is also of the curved surface, and the curved surface is arranged in radian. The so-called radian is a mathematical concept. Two rays 242, 241 are emitted from the center of a circle to form an included angle 244 and an arc opposite to the included angle 244. As can be seen from the definition of the circle, for example, as shown in FIG. 5, the test area is designed in radian, the formed included angle may be 30-80 degrees, and most likely 30, 40, 45, 50, 60 and 70 degrees; and the defined end is designed in radian. Of course, the test area can be connected by arcs with different included angles. Therefore, the highest positions of these curved test areas are slightly higher than the plane of the insertion end, for example, 1-10 mm high, 2-5 mm, and 2-3 mm; such test areas are gradually transformed to the plane from their highest positions, such that the curved test areas enter or depart from the vagina in a comfortable instead of making the test subject feel particularly uncomfortable, and are more comfortable for the test subject.

### Testing device

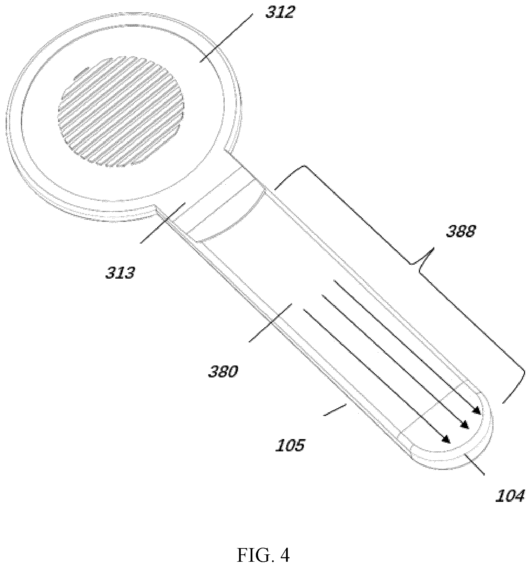

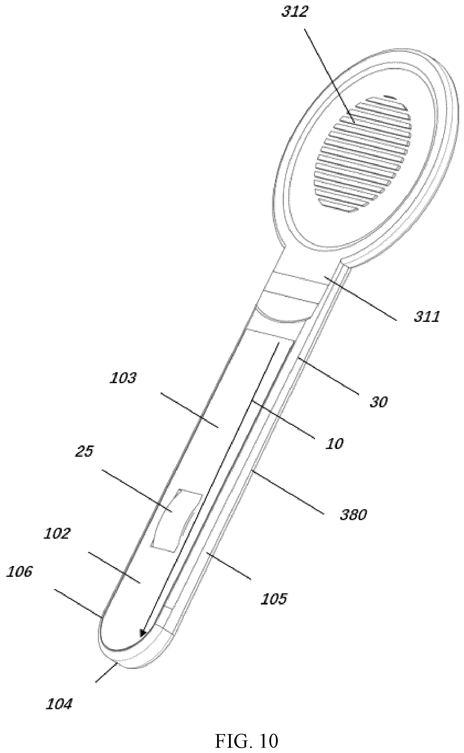

As shown in FIG. 4 and FIG. 10, the testing device has one insertion end 388 that is a long and narrow rod-shaped structure; the insertion end is surrounded by an upper surface 103, left and right surfaces 106, 105, and a front surface 104. These surfaces are all smooth surfaces, and transition between the surfaces is made by the curved surfaces instead of angled surface, for example, the entire device is a cuboid structure (with sharp angles). In this design, when the insertion end 388 enters the vagina to allow the test pad to take samples, operation will be more friendly, without causing uncomfortable feeling, which increases the reliability and trust of the test. In some embodiments, one protruding test area 25 is provided in the plane 103; the test area is designed as a curved surface and gradually bulges from the position of the plane 103 to form the curved surface with a high center and two low sides; as the insertion end 388 is inserted into the vagina, the secretions in the vagina are absorbed mainly by the test pad 25 on the test area 29; after contacting with the secretions, the test pad immediately makes chemical reaction and produces the color change; in case of normal secretions, the test pad does not produce the color change; and the properties of the secretions can be embodied through the color change level. For example, the test pad is yellow at the beginning; if there are the secretions on the test pad, the color of the test pad 29 turns light green or green, indicating that the pH gradually increases; and if the color of the test pad is dark green, the pH is neutral. The color change here is related to the color bottom used, and the test principle can also be similar to that of the pH test strip. In some embodiments, there is a handle 312; the handle is a place to be gripped by hands; the recessed area 311, 313 is arranged between the handle and the insertion end; the insertion end 388 is higher than the gripping location of the handle as a whole; therefore, when the handle is gripped by fingers and the fingers may be located in the recessed area 311, the insertion end 388 instead of the fingers can enter the vagina as much as possible, thereby avoiding contamination of the fingers or discomfort arising from operation.

In some embodiments, the insertion end 388 is composed of an upper card 10 and a lower card 30, a test strip 20 is arranged between the upper card 10 and the lower card 30, and a test area 29 is arranged on the test strip 20, and an absorbent test pad 25 is arranged on the test area. In order to show the bulging or protruding test area, the upper card 10 is provided with one window 101, and the test area bulges from the window 101 and is higher than the surface 102 of the upper card. Therefore, when the surface 102 of the upper card enters the vagina, the test pad on the protruding test area can release secretions, so as to collect and immediately test the samples. In some embodiments, the lower card 30 has a recessed area 310, and insertion holes 301, 302, 303, 304, 305, 306, 308 are provided in the recessed area. Insertion pins or insertion plugs of the upper card are allowed to be inserted into paired insertion holes, such that the upper card and the lower card are fixed together. Correspondingly, the upper card is provided with insertion pins 107, 108, 109, 110, 111, 112, 113, 114, 116. Like a cover, the upper card covers and seals the recessed area 310, such that the insertion pins are inserted into the insertion holes in the recessed area respectively, for example, the insertion pins 107, 108 are inserted into the insertion holes 301, 302 in the recessed area, the insertion pins 109, 110 are inserted into the insertion holes 303, 304, the insertion pins 111, 112 are inserted into the insertion holes 305, 306, and the insertion pin 116 is inserted into the insertion hole 308. In some embodiments, two insertion pins 111, 112 are arranged at two sides of the window 101 and inserted into the insertion holes 305, 306 at two sides of the lug boss 307 arranged on the recessed area 310. Therefore, one lug boss 307 is arranged on the recessed area, and the function of the lug boss is to allow the test area 29 bulge or protrude upward from the window 101, such that the test area is higher than the plane 102 of the upper card. Viewed from the upper card, one protruding structure is provided at the window 101, and the protrusion of the test pad or test area.

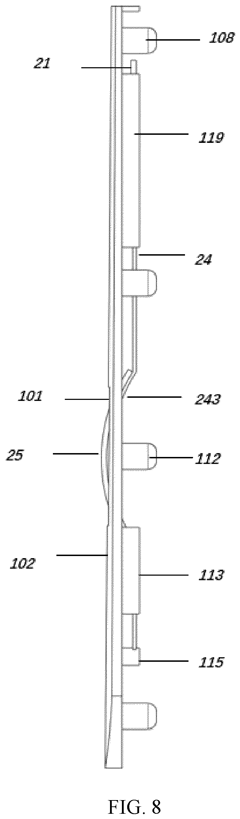

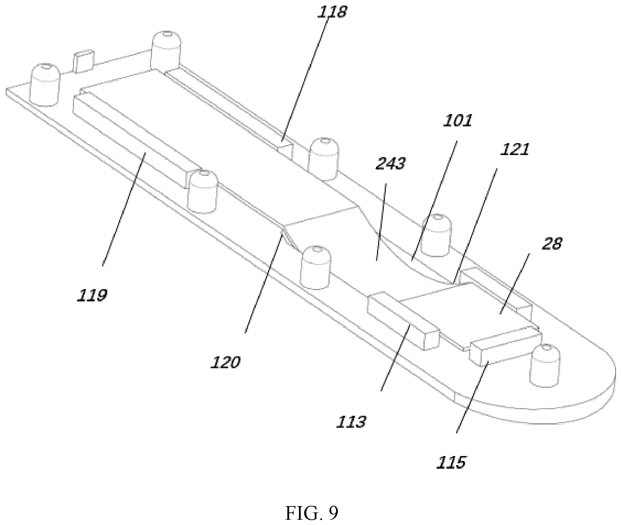

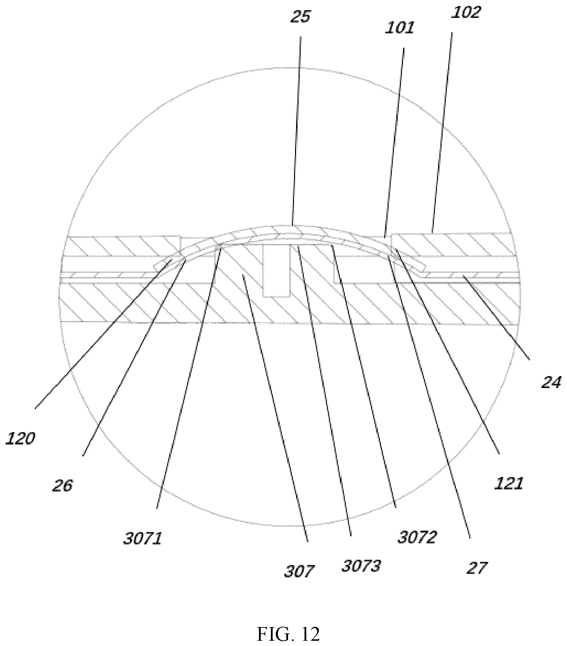

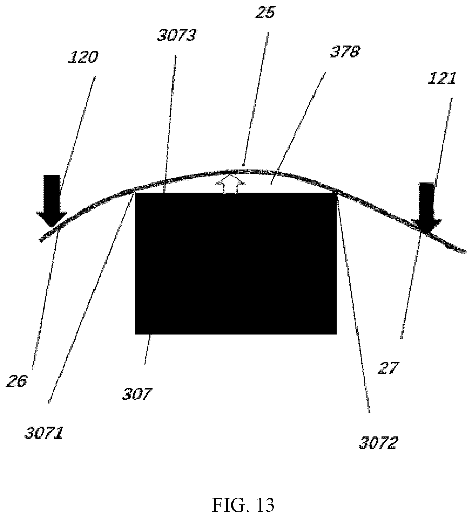

For the stable arrangement of the test strip between the upper card and the lower card and for the convenience of assembly, the upper card is provided with a stop bar 115 for limiting the longitudinal movement of the test strip, and stop bars 113, 114, 118, 119 for limiting the lateral movement of the test strip. The area defined by these stop bars on the upper card 10 is the area of the test strip, in which the test strip 20 is arranged; and when the test strip 20 is pressed, the test area 29 of the test strip 20 is located below the window; and in this case, the test area of the test strip or the test pad 25 is straight instead of being bent. FIG. 8 and FIG. 9 show a schematic diagram of a location of a test strip on an upper card, and a schematic diagram of a structure that a test area is allowed to protrude upward from a window 101 by a lug boss on a recessed area 310. In this case, one end 28 of the test strip is in contact with the stop bar 115 while the test strip is located between the lateral stop bars 118, 119, 113, 114. It can be seen from FIG. 9 that the test area 29 is recessed in the window area when viewed from the back of the upper card 10. If viewed from the front, the test area 29 or the test pad 25 protrudes from the window 101 relative to the plane 102 (FIG. 8). Of course, during the initial assembly, the test strip is straight, and the test area is not naturally curved. In order to fix the test strip onto the upper card, the other end of the test strip (namely, an end far away from the test area) has an adhesive area 21, and the adhesive area and the test pad 25 are located on a same side of the backing or support pad. The adhesive area 21 can be coated with glue or covered with a double-sided adhesive tape, such that the test strip can be bonded to the upper card and specifically bonded to the area defined by the stop bars 119, 118 on the upper card 10, one end of the test strip is fixed, and the other end 28 thereof is free. It is mainly considered that the test area 25 will change from a straight state to a curved state, and the linear length of the test strip will be shortened, such that the free end of the test strip can freely stretch. Of course, in order to fix the end of the test strip, the test strip can also be arranged in the recessed area of the lower card 30 and specifically arranged between jacks, and then the test area is arranged on the lug boss 307. In this case, the end of the test strip can also be fixed in the recessed area, for example, the end of the test strip is fixed between the jacks 301, 302 through the adhesive area 21. This fixing method is not very convenient for the test strip, and it is not as convenient as directly bonding the test strip to the upper card. In order to allow the test area to protrude in a curved surface or radian way when the upper card is installed onto the lower card, the structure as shown in FIG. 12 can be well realized. The lug boss 307 has a plane 3073 that is a right-angled plane. The lug boss has two lateral sides 3071, 3072; the plane 3073 is substantially parallel to or shares a plane with the plane 102 of the upper card, or it doesn't matter if the plane 3073 is slightly lower than the plane 102, such as 1-2 mm. When the adhesive area 21 of the test strip 20 is fixed onto the upper card 10 (for example, the area between the stop bars 119, 118), and the upper card covers the recessed area 310 of the lower card 30 and the insertion pins are inserted into the jacks, the test area 25 is bonded to the backing 243 and is in contact with the surface 3073 of the lug boss 307 on the lower card 30. As the pressure is applied to the upper card and the upper card is fixed with the lower card, the lug boss 307 has an acting force applied to the back of the test area, for example, the acting force is applied to the backing 243; the window 101 of the upper card 10 has two opposite edges 120, 121 that have a thickness, and the longitudinal length of the window 101 is longer than the longitudinal length of the protrusion; therefore, when the upper card 10 covers the recessed area of the lower card 30, a downward pressure is applied to the test area 29 or two ends 26, 27 of the test pad 25 respectively by the opposite edges 120, 121 of the window (a pressure is applied to the end 26 by the edge 120 while a pressure is applied to the end 27 by the edge 121). The positions where the pressure is applied are below the test area 29 and the two sides 3071, 3072 of the lug boss, that is, the positions where the pressure is applied to both ends of the test area by the lateral edges 120, 121 of the window 101 are lower than the positions where the two sides 3071, 3072 of the lug boss are in contact with the back of the test area. When the test area is originally flexible or has certain elasticity, the test area is bent out of the window and bulges (as shown in FIG. 12; FIG. 13 is a schematic diagram of an acting force), such that the middle position of the test area bulges out of the window and does not cover the surface of the lug boss, but has a distance (as indicated by a middle arrow in FIG. 13), for example, a distance of 1-2 mm. Under the acting force, one end of the test strip is fixed, for example, by the adhesive area 21 (a fixing end), while the other end 28 thereof is free and can retract a little distance to be blocked by the stop bar 115. It can be understood that when the test strip is straight, the free end 28 of the test strip slightly exceeds the position of the stop bar 115, and when the test area 29 is bent to have an arc, the retraction of the free end 28 is limited by the stop bar 115. In order to make the test area in a curved state more stably, or to make the test area 29 in a position protruding out of the window for a long time, the insertion holes 305, 306 are arranged in both sides of the lug boss 307, and the insertion pins 111, 112 are arranged on both sides of the window, such that the areas where the window 101 is located are stably combined, and such stable combination enables the edges 102, 121 of the window to exert continuous and steady pressure to the two ends 26, 27 of the test area to keep the test area 25 or the test pad 29 in a bending state. Generally, the validity period of the product is 2 years, and the protruding bending state needs to be kept for at least 2 years. Therefore, only when the insertion end 388 is inserted into the vagina during the test, the protruding test area 29 (for example, the test pad) can release from the liquid sample to successfully complete the test. On the contrary, if the test area does not protrude, but is parallel to the plane where the window is located or recessed at the window, it is difficult for the test area to contact the secretions in the vagina. After all, most vaginal secretions are attached to the superficial skin of the vagina, rather than a liquid sample with a fixed volume. In fact, the secretions on the superficial skin need to be scraped by the test area for testing; therefore, when the insertion end 388 is inserted into the vagina and then removed from the vagina, the test area 25 or the test pad 29 protrudes from the window to conveniently scrape the secretions on the superficial skin of the vagina, and the test area also has other testing functions. In addition, the test area 29 or the test pad 25 is arranged on the backing 243, and the arched shape has a certain distance from the surface of the lug boss and does not completely cover the surface of the lug boss 307, meaning that there is a space 3078 between them; the space can be called a buffer space; the buffer space can make the elastic curved surface move downward or rebound, that is, the test pad 25 with the elastic curved surface can move slightly up and down within the space range from the surface 3073 of the lug boss. Thus, when the insertion end 388 enters the vagina, it is hoped that the test area or the test pad 25 will collect sufficient secretions, and the test area is required to collect secretions without causing damage to the internal surface tissue of the vagina; in this case, the buffer space can play a role in alleviating the damage. In case of obstacles, the test area can deform downward. When the resistance disappears, the test area will restore the original state. Similarly, it can be understood that the buffer space is designed to prevent the test area from directly and completely covering the surface 3073 of the hard lug boss 307. It can be understood that the length of the test pad 25 is greater than the longitudinal length of the window 101, and it is also appropriate that the width of the test pad is generally the same as or smaller than that of the window 101. It can also be understood that the thickness of the test pad on the test area is 1-2 mm, and the support pad 24 is also a 1-2 mm flexible sheet. When the test pad is arched or curved, the curved surface naturally has elasticity.

The reagents on the test pads 29 react with the analyte in the samples and generate a detectable signal showing the test results. These reagents can also react with the liquid samples to determine the properties of the liquid samples (such as pH, specific gravity, or other physical properties). The "reagent" refers to a chemical substance that reacts with the liquid sample and can produce the detectable signal showing the test results. The detectable signal can be compared with that described in a standard. In a specific embodiment, the detectable signal indicates the color change of the reaction pad, and the test result is determined by comparing such color with the color in the standard table or card. The standard table or card indicates positive and/or negative test results by the color, or provides quantitative evaluation associated with the test results. The determination of pH is taken as an example. In a specific embodiment, the reaction pad is beige at the beginning (for example) and turns yellow when reacting with the liquid sample, and the result shows that the pH value is low (acidic). If (for example) the reaction pad reacts with the liquid sample and turns blue, the result shows that the pH value is high (alkaline). The color produced on the reaction pad is between yellow and blue, such as yellow-green or blue-green, which indicates that the result is neutral.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of" and "consisting of' in each embodiment herein may be replaced by the rest 2 terms. The term "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A disposable device comprising a smooth rod body configured to enter into a vagina, wherein a test area is arranged on the smooth rod body, and the test area protrudes from a surface of the smooth rod body, such that when a part of the smooth rod body enters into the vagina, the protruding test area is capable to contact or receive a vaginal secretion to test a property of the vaginal secretion.

2. The testing device according to claim 1, wherein the smooth rod body comprises a chamber, a test strip is provided in the chamber, and the test strip comprises the test area.

3. The testing device according to claim 2, wherein the chamber comprises a lug boss therein, and the test area is allowed to be located on the lug boss in a radian form.

4. The testing device according to claim 3, wherein a window is arranged at a corresponding position of the lug boss, and the test area is higher than a plane where the window is located in a curved surface form from the position of the window.

5. The testing device according to claim 3 or 4, wherein the lug boss is a right-angled cube lug boss and comprises a square lug boss and a cuboid lug boss.

6. The testing device according to any one of claims 1-5, wherein the rod body comprises a lower card and an upper card, the lug boss is arranged on the lower card, the upper card is provided with the window, and the lug boss is located below the window.

7. The testing device according to claim 6, wherein the lower card has a recessed area, the upper card is arranged in the recessed area and covers the recessed area, and the test strip is arranged between the upper card and the lower card.

8. The testing device according to claim 7, wherein a part of the test area is higher than a surface plane of the upper card.

9. The testing device according to any one of claims 6-8, wherein the upper card is provided with a limit block, and the limit block is used for limiting the front, back, left and right positions of the test strip on the upper card, such that the test strip is stably fixed onto the upper card.

10. The testing device according to any one of claims 3-9, wherein a plane of the lug boss along a longitudinal axis substantially shares a height or a plane with a surface of the upper card, an edge of the window is in contact with the test area to give the test area a pressure, and a stress point where the pressure is applied is lower than the plane.

11. The testing device according to claim 10, wherein the plane of the lug boss is a right-angled plane, and a height of the edge of the window is a thickness of the upper card.

12. The testing device according to any one of claims 4-11, wherein a length of the test area is greater than a length of the window in a longitudinal direction.

13. The testing device according to any one of claims 1-12, wherein a chemical reagent is treated on the test area of the test strip, and the chemical reagent is capable to react with the secretion and produce color change and wherein the test area is made of an absorbent material, and a surface of the absorbent material is smooth.

14. The testing device according to claim 13, wherein the absorbent material is arranged on a flexible non-absorbent backing.

15. The testing device according to claim 14, wherein the non-absorbent backing is provided with an adhesive area, and one end of the test strip is fixed into the chamber by the adhesive area and wherein the adhesive area of the test strip is bonded to the upper card, such that one end of the test strip is fixed, and the other end thereof is freely arranged.
